# FASCICULE DE BREVET EUROPEEN

(11) **EP 3 391 810 B1**
(45) Date de publication et mention de la délivrance du brevet: **01.04.2026**
(21) Numéro de dépôt: 17306361.1
(22) Date de dépôt: 10.10.2017
(51) Int. Cl.: A61B 5/00, A61B 1/24

(54) **DISPOSITIF DE PRISE DE VUES DENTAIRE**
VORRICHTUNG ZUR AUFNAHME VON ZAHNBILDERN
DENTAL PHOTOGRAPHY DEVICE

(30) Priorité: 19.04.2017 FR 1753392; 19.04.2017 FR 1753389
(43) Date de publication de la demande: 24.10.2018
(60) Demande divisionnaire: 26153712.0
(73) Titulaire: Dental Monitoring, 75017 Paris (FR)
(72) Inventeur: DEBRAUX, Laurent, 75020 PARIS (FR); SALAH, Philippe, BAGNOLET 93170 (FR); PELLISSARD, Thomas, 92110 CLICHY (FR); GHYSELINCK, Guillaume, 59169 CANTIN (FR)
(74) Mandataire: Ipsilon

(56) Documents cités:
- EP-A2- 1 252 859
- US-A- 4 564 355
- US-A1- 2008 032 252
- US-A1- 2013 209 954

## Description

### Domaine technique

La présente invention concerne un dispositif de prise de vues dentaire, en particulier pour la mise en œuvre d'un procédé tel que décrit dans la demande internationale PCT/EP2015/074896.

### Etat de la technique

PCT/EP2015/074896 décrit un procédé permettant, à partir
- d'un modèle tridimensionnel des dents d'un patient réalisé avant le traitement, dit « modèle de référence initial », puis
- d'une simple image « actualisée » des dents prise pendant le traitement, par exemple d'une photographie prise par le patient,
d'évaluer avec précision le positionnement des dents au moment de l'acquisition de l'image actualisée.

Ce procédé consiste en un processus itératif selon lequel, à chaque itération, des modèles de dents du modèle de référence initial sont déplacés, puis des conditions optimales d'observation du modèle initial ainsi modifié (dit « modèle de référence à tester ») sont déterminées, les conditions optimales d'observation étant définies comme les conditions permettant d'observer le modèle de référence à tester de manière que la vue dudit modèle soit la plus proche possible de l'image actualisée. On teste une succession de « modèles de référence à tester », jusqu'à obtenir un niveau de correspondance maximal entre un modèle de référence à tester et l'image actualisée. Ce dernier modèle de référence à tester est alors considéré comme représentant les dents dans leur position au moment de l'acquisition de l'image actualisée. Idéalement, ce modèle, dit « modèle de référence actualisé », est un modèle de référence tridimensionnel numérique à partir duquel l'image actualisée aurait pu être prise si ce modèle avait été réel. En pratique, il représente effectivement, avec une grande précision, les dents dans leur position au moment de l'acquisition de l'image actualisée.

Généralement plusieurs images actualisées sont nécessaires pour évaluer le positionnement de plusieurs dents. Pour chaque image actualisée, le procédé doit être mis en œuvre. Le procédé décrit dans la demande internationale PCT/EP2015/074896 peut donc être long à mettre en œuvre.

Par ailleurs, les images actualisées peuvent être utilisées pour détecter des évolutions de l'aspect des dents ou des tissus mous comme les gencives, et en particulier de leur couleur et de leur translucidité. La comparaison de différentes photos ne fournit cependant pas toujours des résultats satisfaisants.

Un objectif de la présente invention est de répondre, au moins partiellement, à ces problèmes.

Le document US-A-2013/209 954 décrit un support d'image de cavité buccale comprenant un embout buccal et un support de caméra, la caméra étant un téléphone portable avec appareil photo intégré et processeur embarqué. Le support de caméra comprend en outre un chemin optique et un clip.

### Résumé de l'invention

L'invention propose un kit de prise de vues selon la revendication 1.

Comme cela apparaîtra plus clairement dans la suite de la description, un kit selon l'invention permet avantageusement de définir un positionnement précis de l'appareil d'acquisition d'images par rapport à l'écarteur dentaire. En particulier, la distance entre les moyens de fixation de l'appareil d'acquisition d'images et l'écarteur dentaire peut être prédéterminée, ce qui permet d'accélérer le réglage de l'appareil d'acquisition d'images. En outre, l'orientation de l'appareil d'acquisition d'images par rapport au support peut être prédéterminée, ce qui permet de prévoir approximativement la représentation de l'écarteur dentaire, et, à travers l'écarteur dentaire, des arcades du patient, sur les images actualisées acquises au moyen de l'appareil d'acquisition d'images.

Le traitement ultérieur des images, en particulier suivant le procédé de PCT/EP/2015/074896, en est accéléré.

Suivant un premier perfectionnement principal, le dispositif comporte un miroir fixé sur le support et lesdits moyens de fixation sont configurés pour une fixation de l'appareil d'acquisition d'images sur le support dans une position dans laquelle l'appareil d'acquisition est orienté pour recevoir une image composée comportant une image directe de l'ouverture d'écarteur et une image de l'ouverture d'écarteur réfléchie par le miroir.

Dans une position de service dans laquelle l'écarteur est disposé sur la bouche du patient et l'appareil d'acquisition d'images est fixé sur le support par lesdits moyens de fixation, ledit appareil d'acquisition voit ainsi une image composée comportant une image directe des dents observée directement à travers l'ouverture d'écarteur et une image réfléchie des dents renvoyée par le miroir.

Comme cela apparaîtra plus clairement dans la suite de la description, un dispositif selon le premier perfectionnement principal de l'invention permet ainsi d'acquérir simultanément plusieurs images des dents, observées selon des angles différents. La procédure d'acquisition en est accélérée.

En outre, la détermination des conditions d'acquisition de l'image directe, ou de l'image réfléchie, permet la détermination des conditions d'acquisition de l'image réfléchie, ou de l'image directe respectivement. Il suffit en effet de connaître l'orientation du miroir et sa position par rapport à l'écarteur et à l'appareil d'acquisition pour déterminer le positionnement dans l'espace de l'appareil d'acquisition qui aurait permis, en l'absence du miroir, d'observer l'image réfléchie. La mise en œuvre d'un procédé tel que celui dans la demande internationale PCT/EP2015/074896 en est dès lors considérablement accélérée.

En effet, typiquement, le patient prend une photo de ses dents depuis la droite, une photo de ses dents depuis la gauche et une photo de ses dents vues de face. Mais le logiciel mettant en œuvre le procédé décrit dans la demande internationale PCT/EP2015/074896 ignore la position de l'appareil d'acquisition à ces différentes étapes et ne peut la déduire d'un traitement des autres images. Il doit donc, à chaque fois, la retrouver pour définir les conditions optimales d'observation.

Suivant un deuxième perfectionnement principal, le dispositif comporte
- une mire colorimétrique et/ou une mire de translucidité, de préférence une mire colorimétrique et une mire de translucidité, fixée(s) de préférence sur le support ; et
- de préférence une source lumineuse orientée de manière à éclairer d'une part les dents du patient à travers l'ouverture d'écarteur et d'autre part ladite mire colorimétrique et/ou ladite mire de translucidité,
lesdits moyens de fixation de l'appareil d'acquisition étant configurés pour immobiliser l'appareil d'acquisition dans une position dans laquelle il est orienté pour recevoir une image de l'ouverture d'écarteur d'une part et de ladite mire colorimétrique et/ou de ladite mire de translucidité d'autre part.

Comme cela apparaîtra plus clairement dans la suite de la description, un dispositif selon le deuxième perfectionnement principal de l'invention permet ainsi de déterminer avec précision la couleur et la translucidité des dents et/ou des tissus mous comme les gencives. En particulier, l'éclairage peut être contrôlé de manière à limiter les perturbations par l'environnement lumineux du dispositif. Différentes images prises à des instants différents peuvent donc être comparées avec une bonne précision.

Suivant un troisième perfectionnement principal, le support présente la forme d'un boîtier ne débouchant vers l'extérieur sensiblement que par l'ouverture d'écarteur et par une ouverture d'acquisition à travers laquelle l'appareil d'acquisition fixé sur le support reçoit au moins une image de l'ouverture d'écarteur.

Ainsi, le support définit une chambre fermée lorsque l'ouverture de l'écarteur et l'ouverture d'acquisition sont obturées.

Avantageusement, l'intérieur de la chambre ne reçoit donc sensiblement pas de rayons lumineux de l'extérieur, ce qui facilite le contrôle de l'éclairage lors de l'acquisition d'images actualisées. Un boîtier fermé préserve également l'intimité du patient.

Bien entendu, les caractéristiques des différents perfectionnements principaux de l'invention peuvent être combinées.

Quel que soit le perfectionnement principal considéré, un dispositif selon l'invention présente de préférence une ou plusieurs des caractéristiques optionnelles suivantes :
- le support définit une chambre débouchant vers l'extérieur par l'ouverture d'écarteur et par une ouverture d'acquisition à travers laquelle l'appareil d'acquisition fixé par lesdits moyens de fixation reçoit ladite image composée ;
- le support est télescopique de manière que la distance entre les ouvertures d'écarteur et d'acquisition soit variable ;
- le dispositif comporte au moins deux dits miroirs orientés perpendiculairement l'un à l'autre et renvoyant chacun une image réfléchie de l'ouverture d'écarteur, chacune desdites images réfléchies étant représentée sur ladite image composée ;
- les moyens de fixation de l'appareil d'acquisition sur le support sont désactivables ;
- les moyens de fixation de l'appareil d'acquisition et/ou de l'écarteur sur le support sont choisis dans le groupe constitué par un clips, une bande auto-agrippante, des mâchoires de serrage, une vis, un aimant, un capot et une forme complémentaire entre le support et l'appareil d'acquisition ;
- le dispositif comporte des moyens de fixation de l'écarteur sur le support désactivables, l'écarteur pouvant ainsi être fixé de manière amovible sur le support ;
- l'écarteur comporte des pattes qui, dans une position montée de l'écarteur sur le support, sont insérées dans des logements profilés respectifs du support, chaque logement présentant une section transversale de forme générale en U, l'ouverture du U débouchant de préférence vers le haut ou vers le bas dans une position de service dans laquelle l'écarteur est disposé sur la bouche d'un patient maintenant sa tête verticale ;
- dans ladite position montée, l'écarteur est maintenu en flexion, en appui élastique sur le support ;
- le support comporte un ou plusieurs, de préférence deux crochets configurés pour recevoir l'écarteur dans ladite position montée ;
- la source lumineuse est configurée de manière à projeter un repère vers l'ouverture d'écarteur ;
- le dispositif comporte un module de contrôle configuré pour contrôler les propriétés du rayonnement émis par la source lumineuse, de préférence en fonction du rayonnement lumineux reçu par l'ouverture d'écarteur ;
- le module de contrôle est configuré pour commander la source lumineuse pour que plus de 50%, plus de 70%, plus de 90%, voire sensiblement 100% de l'intensité du rayonnement reçu par l'ouverture d'écarteur proviennent de la source lumineuse ;
- le dispositif comporte un module de traitement dans lequel sont enregistrées les propriétés de couleur et de translucidité réelles des mires colorimétrique et de translucidité, respectivement, le module de traitement comprenant des instructions de code de programme pour corriger une image représentant lesdites mires de manière que les représentations desdites mires sur l'image présentent lesdites propriétés de couleur et de translucidité.

Un programme d'ordinateur, ou « applicatif », et en particulier un applicatif spécialisé pour téléphone mobile est aussi décrit, , comprenant des instructions de code de programme pour guider une prise de vues, et en particulier, de préférence, pour
- préciser à un opérateur le nombre d'images actualisées qu'il doit acquérir, et/ou
- guider un opérateur lors d'un réglage de la géométrie du support et/ou d'une orientation du miroir et/ou d'un positionnement de la bouche du patient sur l'écarteur, et/ou
- pour commander un ou plusieurs actionneurs aptes à modifier ladite géométrie, notamment modifier la longueur du support, et/ou ladite orientation du miroir,

- un support informatique sur lequel est enregistré un tel programme, par exemple une mémoire ou un CD-ROM, et
- un appareil personnel, en particulier un téléphone mobile ou une tablette, dans lequel est chargé un tel programme.

Selon le premier perfectionnement principal de l'invention, l'appareil d'acquisition d'images peut ainsi acquérir ladite image composée.

Un kit selon l'invention présente de préférence une ou plusieurs des caractéristiques optionnelles suivantes :
- l'appareil d'acquisition d'images comporte un programme d'ordinateur;
- l'appareil d'acquisition d'images est configuré pour prendre successivement plusieurs photos, avec des distances focales différentes, en conséquence d'un unique déclenchement.

Dans un mode de réalisation préféré, le dispositif de prise de vues comporte un organe de détection et l'appareil d'acquisition d'images comporte un détecteur de l'organe de détection configuré de manière à détecter l'organe de détection lorsque l'organe de détection est à moins de 20 cm, de préférence à moins de 10 cm, de préférence à moins de 5 cm et/ou de préférence à plus de 1 cm du dispositif de prise de vues.

De préférence, le détecteur de l'organe de détection est configuré de manière à ne détecter l'organe de détection que lorsque l'organe de détection est à moins de 100 cm, de préférence à moins de 50 cm, de préférence à moins de 30 cm, de préférence à moins de 20 cm, de préférence à moins de 10 cm, de préférence à moins de 5 cm du dispositif de prise de vues.

Comme cela apparaîtra plus clairement dans la suite de la description, un tel kit de prise de vues permet avantageusement à l'appareil d'acquisition d'images de réagir à l'approche du dispositif de prise de vues, et en particulier de lancer un programme d'ordinateur apte à guider ladite prise de vue.

### Définitions

Un « perfectionnement principal de l'invention » définit une caractéristique optionnelle particulièrement remarquable.

Par « patient », on entend toute personne pour laquelle un dispositif selon l'invention peut être mis en œuvre, que cette personne soit malade ou non, ou que cette personne soit en cours de traitement ou non. Un dispositif selon l'invention peut être utilisé pour un autre animal qu'un être humain.

On appelle "téléphone mobile" un appareil de moins de 500 g, doté d'un capteur lui permettant de capturer des images, capable d'échanger des données avec un autre appareil éloigné de plus de 500 km du téléphone mobile, et capable d'afficher lesdites données, et notamment lesdites images.

Les « conditions d'acquisition » d'une image des dents précisent la position et l'orientation dans l'espace d'un appareil d'acquisition d'images relativement aux dents du patient, et de préférence la calibration de cet appareil d'acquisition d'images, pour acquérir à l'instant de ladite acquisition, par observation directe, ladite image. Les conditions d'acquisition d'une image réfléchie précisent donc la position et l'orientation dans l'espace que devrait prendre l'appareil d'acquisition d'images, en l'absence du miroir, pour acquérir l'image réfléchie.

La « calibration » d'un appareil d'acquisition est constituée par l'ensemble des valeurs des paramètres de calibration. Un paramètre de calibration est un paramètre intrinsèque à l'appareil d'acquisition (à la différence de sa position et de son orientation) dont la valeur influence l'image acquise. Par exemple, l'ouverture de diaphragme est un paramètre de calibration qui modifie la profondeur de champ. Le temps d'exposition est un paramètre de calibration qui modifie la luminosité (ou « l'exposition ») de l'image. La distance focale est un paramètre de calibration qui modifie l'angle de vue, c'est-à-dire le degré de « zoom ». La « sensibilité » est un paramètre de calibration qui modifie la réaction du capteur d'un appareil d'acquisition numérique à la lumière incidente.

De préférence, les paramètres de calibration sont choisis dans le groupe formé par l'ouverture de diaphragme, le temps d'exposition, la distance focale et la sensibilité.

Par "image", on entend une image en deux dimensions, comme une photographie. Une image est formée de pixels.

Par « image actualisée », on entend une image acquise au moyen d'un appareil d'acquisition d'images. Une image composée, suivant le premier perfectionnement principal de l'invention, est un exemple d'image actualisée.

« Fixer » signifie « solidariser de manière rigide ». « Fixer » ne signifie pas nécessairement « solidariser de manière définitive ».

"Comprendre", "comporter" et "présenter" doivent être interprétés de manière large et non limitative, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente, en perspective, un dispositif selon l'invention, observé par transparence du côté de l'écarteur ;
- la figure 2 représente, en perspective, un dispositif selon l'invention représenté du côté de l'ouverture d'acquisition ;
- la figure 3 représente un exemple d'image composée ;
- les figures 4a, 4bet 4c représentent le dispositif de la figure 1 vu de dessus, vu du côté de l'ouverture d'acquisition et vu du côté de l'ouverture d'écarteur, respectivement ;
- les figures 5a et 5b représentent le dispositif de la figure 2 dans des positions rétractée et déployée du support, respectivement ;
- les figures 6a, 6b et 6c représentent en perspective (figures 6a et 6b) et vue de dessus (figure 6c) la partie du support d'un dispositif selon l'invention sur laquelle un écarteur peut être monté, comme représenté sur la figure 6b ; et
- la figure 7 représente un kit selon l'invention comportant un dispositif de prise de vues selon l'invention vu de côté.

Dans les différentes figures, les organes identiques ou analogues ont été désignés avec des références identiques.

### Description détaillée

### Dispositif

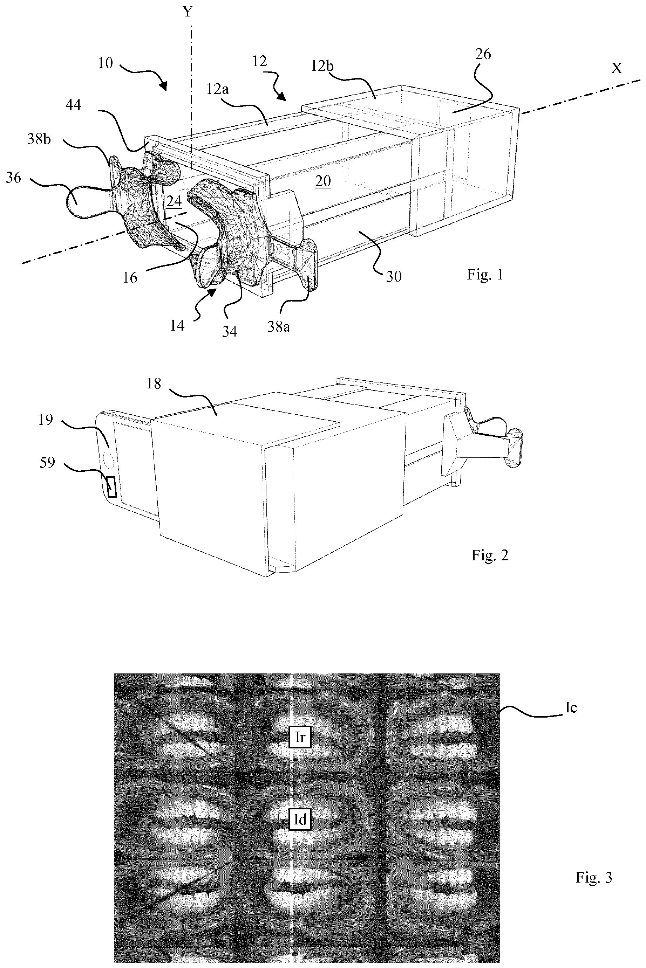

Le dispositif de prise de vues 10 représenté sur la figure 1 comporte un support 12, sous la forme d'un boîtier, optionnellement télescopique, un écarteur dentaire 14, de préférence au moins un miroir 16, et des moyens de fixation 18 d'un appareil d'acquisition d'images 19, représentés sur la figure 2.

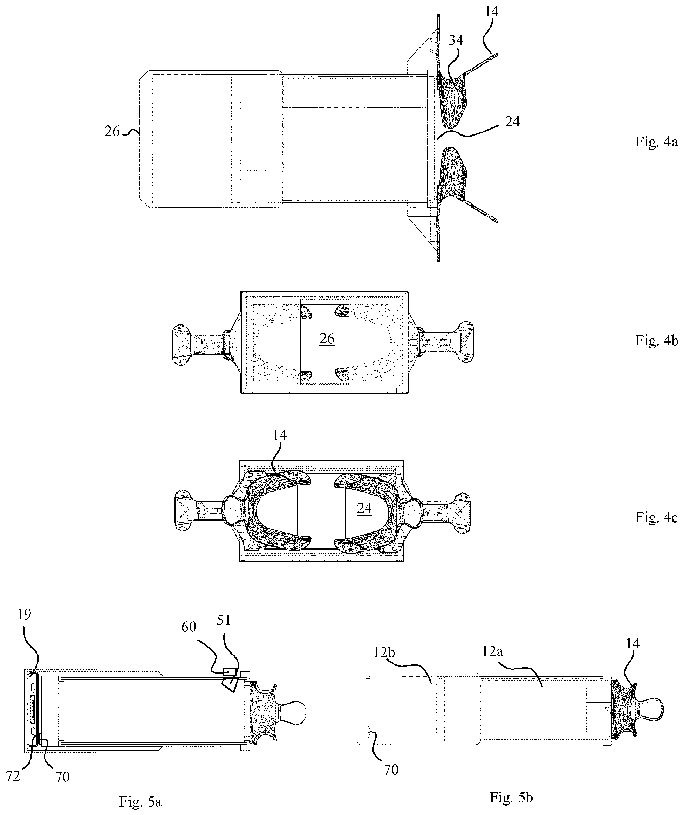

Dans un mode de réalisation, le support 12 comporte une partie mâle 12a et une partie femelle 12b montées coulissantes l'une dans l'autre, suivant un axe d'écarteur X, entre des positions rétractée (figure 5a) et déployée (figure 5b).

Dans un mode de réalisation, une échelle est disposée sur la partie mâle 12a du support. De préférence, cette échelle fournit des indications facilitant le réglage de la longueur, selon l'axe X, du support 12, par exemple en portant une marque pour chaque type d'appareil d'acquisition d'images.

Le support 12 définit une chambre 20 dont la longueur selon l'axe X dépend de la position relative des parties mâle et femelle du support 12 lorsque le boîtier est télescopique, ou est constante.

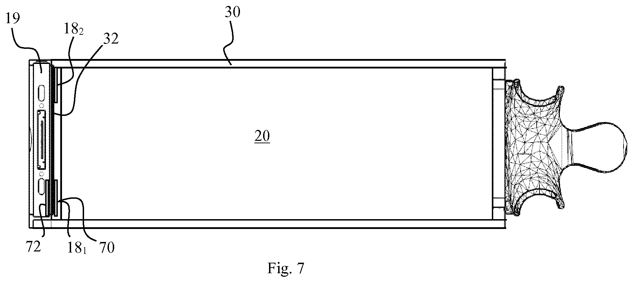

Dans un mode de réalisation préféré, le boîtier n'est pas télescopique (figure 7). Lorsque le boîtier n'est pas télescopique, la distance entre les ouvertures d'écarteur et d'acquisition est avantageusement constante. L'analyse des images actualisées en est facilitée. En outre, ce mode de réalisation est avantageusement simple, peu coûteux et ergonomique.

Dans le mode de réalisation représenté, la chambre 20 débouche vers l'extérieur sur deux faces d'extrémité opposées du support 12, par une ouverture d'écarteur 24 et une ouverture d'acquisition 26, respectivement.

La paroi latérale 30 du support 12, qui s'étend entre les deux faces d'extrémité, est de préférence sensiblement cylindrique d'axe X, de préférence de section rectangulaire.

Dans un mode de réalisation, le support 12 est pourvu d'une fenêtre à travers laquelle la lumière de l'environnement peut pénétrer à l'intérieur de la chambre 20 pour éclairer les dents.

De préférence, la chambre 20 ne débouche cependant vers l'extérieur que par les ouvertures d'écarteur et d'acquisition.

Le boîtier ainsi constitué peut être par exemple en plastique ou en carton.

Les moyens de fixation 18 sont configurés de manière que l'appareil d'acquisition d'images puisse être fixé dans une position d'acquisition dans laquelle son objectif fait face à l'ouverture d'acquisition 26, voire obture l'ouverture d'acquisition 26.

Les moyens de fixation 18 de l'appareil d'acquisition sur le support 12 peuvent être quelconques. De préférence, ils permettent une fixation rigide. De préférence, ils sont désactivables de manière réversible, c'est-à-dire que l'utilisateur peut à volonté solidariser et désolidariser l'appareil d'acquisition du support.

De préférence, les moyens de fixation 18 de l'appareil d'acquisition sont choisis dans le groupe constitué par des moyens de clipsage, des bandes auto-agrippantes de type Velcro^{®}, des mâchoires de serrage, des vis, des aimants, et une complémentarité de forme entre le support et l'appareil d'acquisition. Dans le mode de réalisation représenté sur la figure 2, les moyens de fixation 18 sont constitués par un capot qui peut être serré contre le support 12.

Dans un mode de réalisation, les moyens de fixation 18 de l'appareil d'acquisition sont adaptés pour la fixation d'un appareil photo conventionnel, par exemple de type reflex.

De préférence, comme représenté sur la figure 7, les moyens de fixation 18 de l'appareil d'acquisition sont magnétiques. En particulier, un ou plusieurs aimants 18₁, 18₂ peuvent être prévus sur le support et une ou plusieurs pièces métalliques peuvent être fixées sur l'appareil d'acquisition d'images de manière à coopérer avec le ou les aimants. En particulier, une plaque métallique 32 peut être immobilisée sur l'appareil d'acquisition d'images, de préférence prise en sandwich entre l'appareil d'acquisition et une coque de protection fixée sur l'appareil d'acquisition.

Dans un mode de réalisation, l'appareil d'acquisition comporte plusieurs pièces métalliques et/ou le support comporte plusieurs aimants.

Avantageusement, il suffit donc que l'opérateur approche l'appareil d'acquisition du support pour que l'appareil d'acquisition se fixe sur le support. L'utilisation d'une fixation magnétique permet également une fixation fiable et très précise, même lorsque l'opérateur ne regarde pas le support ou l'appareil d'acquisition.

De préférence, les moyens de fixation 18 de l'appareil d'acquisition, et en particulier les aimant(s) et pièce(s) métallique(s), sont configurés de manière que l'appareil d'acquisition ne puisse être fixé sur le support que dans une unique position prédéterminée. La fixation de l'appareil d'acquisition ne nécessite donc aucune formation particulière de l'opérateur. Le cas échéant, des guides visuels peuvent être présent pour guider l'opérateur et s'assurer du bon positionnement de l'appareil d'acquisition.

L'écarteur 14 peut présenter les caractéristiques des écarteurs conventionnels. Il comporte un rebord 34 s'étendant autour de l'ouverture d'écarteur 24 et agencé de manière que les lèvres du patient puissent y reposer en laissant apparaître les dents du patient à travers ladite ouverture d'écarteur. Dans le mode de réalisation représenté, l'écarteur 14 comporte également des oreilles 36 agencées de manière à écarter les joues des dents et des pattes droite 38a et gauche 38b, sensiblement perpendiculaires à l'axe X, facilitant sa manipulation.

L'écarteur 14 est de préférence en un matériau biocompatible, par exemple en matière plastique.

L'écarteur 14 peut être venu de matière avec le support 12 ou être fixé, de préférence rigidement, sur le support 12 par tout moyen.

De préférence, l'écarteur est amovible, c'est-à-dire qu'il peut être monté et démonté du support par l'opérateur. Avantageusement, le même support peut donc servir pour plusieurs écarteurs, et en particulier pour plusieurs écarteurs de tailles différentes.

Les moyens de fixation 15 de l'écarteur sur le support peuvent être par exemple des moyens de clipsage, des bandes auto-agrippantes de type Velcro^{®}, des mâchoires de serrage, des vis, des aimants, et une complémentarité de forme entre le support et l'écarteur.

Dans un mode de réalisation, l'écarteur 14 est fixé au moyen d'une insertion d'une des pattes droite 38a et gauche 38b dans un logement du support, puis d'un clipsage de l'autre patte sur le support.

Dans un mode de réalisation, l'écarteur 14 est fixé au moyen d'un clipsage des pattes dans des logements du support respectifs.

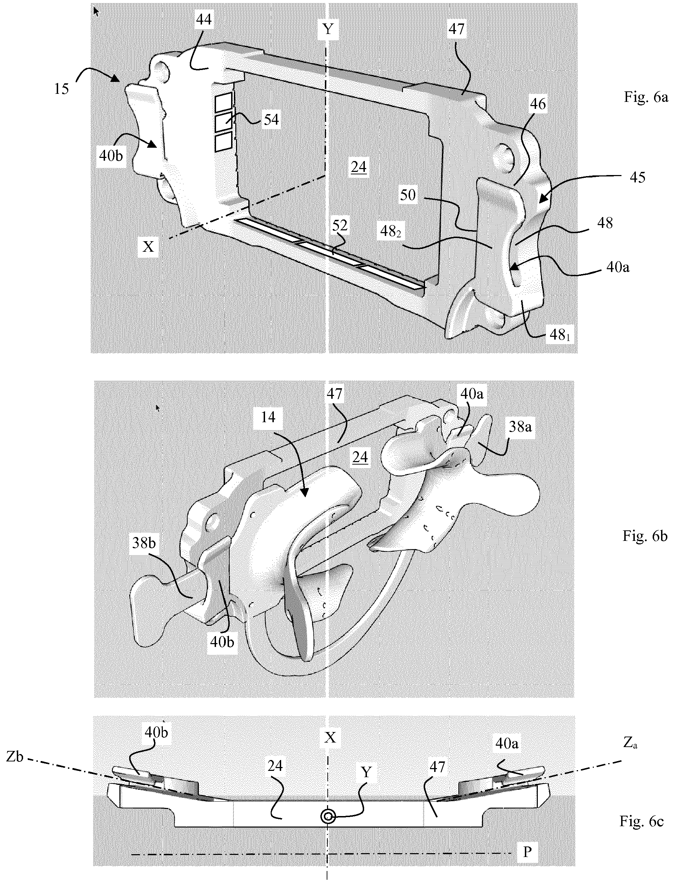

Dans un mode de réalisation préféré, illustré par la figure 6, les moyens de fixation 15 de l'écarteur sur le support comportent un crochet sur lequel l'écarteur 14 peut être accroché. Les moyens de fixation 15 de l'écarteur sur le support comportent de préférence un crochet droit 40a et un crochet gauche 40b agencés pour recevoir les pattes transversales droite 38a et gauche 38b de l'écarteur, respectivement. Les crochets droit et gauche étant similaires, seul un crochet est décrit en détail ci-après.

De préférence, ledit crochet fait saillie de la face arrière 44 du support, sensiblement perpendiculaire à l'axe X, comme représenté sur la figure 1. La face arrière 44 est de préférence définie par une plaque 47 (figure 6a) fixée sur un chant de la paroi latérale 30 du support 12.

De préférence, le crochet présente la forme d'une cornière 45. La cornière comporte de préférence des première et deuxième ailes perpendiculaires l'une à l'autre. La première aile 48₁, fixée sur la face arrière 44 du support, est de préférence sensiblement parallèle à l'axe X et la deuxième aile 48₂ est de préférence sensiblement perpendiculaire à l'axe X et, de préférence, s'étend vers le haut depuis la première aile. Avec la face arrière 44 du support, la cornière 45 définit un logement profilé en U dont l'ouverture principale 46, orientée vers le haut, est dimensionnée pour recevoir une patte de l'écarteur. De préférence encore, le logement profilé débouche, à ses deux extrémités droite et gauche, par des ouvertures droite 48 et gauche 50 à travers lesquelles une patte de l'écarteur peut être glissée dans le crochet, c'est-à-dire entre la face arrière 44 du support et la deuxième aile 48₂ de la cornière 45, jusqu'à entrer en butée avec la première aile 48₁.

De préférence, les crochets sont configurés de manière que l'écarteur ne puisse y être inséré qu'en force, de préférence par ouverture élastique des crochets.

De préférence, les crochets sont configurés de manière que l'écarteur ne puisse y être inséré sans être déformé, de préférence par flexion autour d'un axe Y sensiblement perpendiculaire à l'axe X, de préférence sensiblement vertical dans la position de service.

De préférence encore, dans la position montée de l'écarteur sur le support, les moyens de fixation 15 maintiennent l'écarteur fléchi autour de l'axe Y.

Dans le mode de réalisation de la figure 6 (figure 6c), les axes Zₐ et Z_{b} des cornières des crochets droit et gauche sont ainsi inclinés par rapport à un plan transversal P perpendiculaire à l'axe X, alors que les pattes droite et gauche de l'écarteur sont sensiblement coplanaires lorsque l'écarteur est au repos, désolidarisé du support. La largeur des ouvertures principales 46 des crochets droit et gauche est sensiblement identique à l'épaisseur des pattes droite et gauche de l'écarteur, respectivement, ce qui oblige l'opérateur à fléchir l'écarteur autour de l'axe Y pour insérer ces pattes dans ces crochets. La forme des crochets empêche ensuite un retour de l'écarteur dans sa position de repos.

L'appui élastique de l'écarteur sur le support ainsi obtenu favorise avantageusement son maintien en position.

Dans un mode de réalisation préféré, le dispositif comporte une source lumineuse 51, de préférence orientée vers l'ouverture d'écarteur 24 (fig. 5a), de manière à éclairer les dents du patient à travers l'ouverture d'écarteur 24. La source lumineuse 51 peut en particulier émettre une lumière blanche, une lumière monochrome, un rayonnement infrarouge ou, de préférence, un rayonnement ultraviolet.

La source lumineuse 51 peut être un flash.

La source lumineuse est de préférence fixée sur le boîtier. Elle comprend de préférence des LED.

Dans un mode de réalisation, la source lumineuse 51 est configurée de manière à projeter sur les dents, à travers l'ouverture d'écarteur 24, un repère, de préférence une grille laser. Avantageusement, la représentation d'un repère sur les images facilite la détermination de la forme des dents. Une première estimation de la forme des dents est ainsi possible sans mettre en œuvre le procédé décrit dans PCT/EP2015/074896.

Dans un mode de réalisation, un filetage est ménagé sur la paroi latérale 30 du boîtier, de préférence sur la paroi latérale de la partie femelle 12b du support. Le filetage est de préférence conformé de manière à autoriser la fixation du support sur un trépied pour appareil photographique.

Dans un mode de réalisation, le dispositif comporte encore un capot d'acquisition et un capot d'écarteur, conformés pour sélectivement obturer l'ouverture d'acquisition et l'ouverture d'écarteur pour faciliter le stockage du dispositif. De préférence, le capot d'écarteur est conformé pour sélectivement obturer l'ouverture d'écarteur après démontage de l'écarteur.

**Suivant le premier perfectionnement principal de l'invention,** le dispositif comporte un miroir 16.

Le miroir 16 est fixé sur la face intérieure de la paroi latérale 30.

Le miroir 16 est de préférence plan.

Le nombre et la forme des miroirs ne sont pas limitatifs. En particulier, le miroir peut être de forme rectangulaire, sphérique, ovale, octogonale ou hexagonale.

Dans le mode de réalisation représenté, des miroirs 16 tapissent toute la face intérieure de la paroi latérale de la chambre 20, au moins dans la partie femelle 12b du support 12 et, de préférence, également dans la partie mâle 12a du support.

Dans un mode de réalisation, le dispositif comporte quatre miroirs disposés chacun sur une des quatre faces de la paroi latérale du support, et en particulier de la partie femelle 12b et, de préférence, de la partie mâle 12a du support. Dans un mode de réalisation préféré, chaque miroir couvre entièrement la face du support 12 sur laquelle il s'étend.

La longueur d'un miroir peut être supérieure à 3 cm, supérieure à 5 cm et/ou inférieure à 30 cm, inférieure à 20 cm, inférieure à 15 cm, ou inférieure à 10 cm. La largeur d'un miroir peut être supérieure à 2 cm, supérieur à 3 cm et/ou inférieure à 10 cm ou inférieure à 8 cm.

Dans le mode de réalisation représenté, les miroirs sont fixés sur le support, de préférence de manière définitive, par exemple au moyen d'une colle.

Le miroir 16, de préférence chaque miroir 16, s'étend de préférence parallèlement à l'axe X. Dans le mode de réalisation représenté, les miroirs 16 s'étendent perpendiculairement les uns aux autres, deux à deux.

La présence simultanée d'un miroir et d'une source lumineuse 51 est particulièrement avantageuse puisqu'elle permet d'acquérir des images des dents faisant apparaitre simultanément le reflet de la source lumineuse 51 sur les dents, mais également le reflet de la source lumineuse fictive obtenue par la réflexion de la source lumineuse 51 par le miroir. L'analyse de la position relative de ces reflets dans les images permet avantageusement de déterminer grossièrement l'orientation de la surface des dents les renvoyant, ainsi que la position de cette surface par rapport à l'appareil d'acquisition.

**Suivant le deuxième perfectionnement principal de l'invention,** le dispositif comporte une mire colorimétrique 52 et/ou une mire de translucidité 54 est fixée sur le support, de préférence dans la chambre 20, ou sur le capot d'écarteur, ou sur l'écarteur dentaire.

Le nombre et la forme des mires ne sont pas limitatifs. Dans un mode de réalisation de la figure 6a, le support porte par exemple trois mires colorimétriques 52 et trois mires de translucidité 54.

Avantageusement, les mires colorimétrique(s) 52 et de translucidité 54 permettent, pour chaque image, de corriger les erreurs de teinte propres à chaque appareil d'acquisition d'images.

Les mires colorimétrique(s) 52 et de translucidité 54 permettent également avantageusement de déterminer les couleurs et la translucidité exactes des dents ou des gencives, ce qui permet de détecter toute évolution de ces propriétés.

De préférence, les mires colorimétrique(s) 52 et de translucidité 54 sont fixées à proximité de l'ouverture d'écarteur, de préférence à moins de 5 cm, moins de 3 cm, moins de 1 cm de l'ouverture d'écarteur. De préférence, les mires colorimétrique(s) 52 et de translucidité 54 sont fixées sensiblement dans le plan de l'ouverture d'écarteur, comme représenté sur la figure 6a.

De préférence, le module de contrôle 60 contrôle les propriétés du rayonnement émis par la source lumineuse 51, de préférence en fonction du rayonnement lumineux reçu par l'ouverture d'écarteur. Un capteur de lumière peut être prévu, à proximité de l'ouverture d'écarteur, pour évaluer le rayonnement lumineux reçu par ladite ouverture d'écarteur.

Dans un mode de réalisation, le module de contrôle 60 contrôle la puissance de la source lumineuse 51 pour que plus de 50%, plus de 70%, plus de 90%, voire sensiblement 100% de l'intensité du rayonnement reçu par l'ouverture d'écarteur proviennent de la source lumineuse 51.

**Suivant le troisième perfectionnement principal de l'invention,** le support présente la forme d'un boîtier qui ne débouche vers l'extérieur sensiblement que par les ouvertures d'écarteur et d'acquisition. Avantageusement, l'influence de l'environnement extérieur au boîtier est donc limitée, ce qui permet une acquisition d'images dans des conditions constantes. Par ailleurs, lorsque le dispositif comporte une source lumineuse 51, un boîtier fermé permet avantageusement de limiter la puissance de la source lumineuse 51.

De préférence, la surface totale des ouvertures autres que les ouvertures d'écarteur et d'acquisition représente moins de 10%, de préférence moins de 5%, de préférence moins de 1% de la surface de la paroi latérale du boîtier délimitant la chambre 20. De préférence, le boîtier ne comporte pas de telles « autres ouvertures ».

Dans un mode de réalisation, la paroi latérale délimitant la chambre 20 est conformée ou constituée en un matériau ne permettant pas de distinguer précisément le contenu de la chambre 20, ni les arcades du patient lorsque le dispositif est dans une position de service dans laquelle un patient a disposé ses lèvres dans les goulottes de l'écarteur. Avantageusement, une telle paroi latérale permet en effet de parfaitement préserver l'intimité de l'utilisateur lors de l'acquisition des images actualisées.

Dans un mode de réalisation, la paroi latérale est translucide.

De préférence, la paroi latérale est opaque. Avantageusement, le volume intérieur de la chambre ne reçoit donc sensiblement pas de lumière de l'extérieur, ce qui garantit des conditions d'acquisition d'images actualisées constantes.

### Kit de prise de vues

Un kit de prise de vues selon l'invention comporte un dispositif de prise de vues selon l'invention et un appareil d'acquisition d'images 19.

L'appareil d'acquisition d'images 19 fournit de préférence des images en couleurs, et/ou des images infrarouges. Les images infrarouges permettent avantageusement de faire apparaître les dents avec un excellent contraste.

De préférence, l'appareil d'acquisition d'images 19 est un appareil personnel couramment disponible dans le commerce, par exemple un téléphone mobile, un appareil photo dit « connecté », une montre dite « intelligente », ou « smartwatch », ou une tablette ou un ordinateur personnel, fixe ou portable, comportant un système d'acquisition d'images, comme une webcam ou un appareil photo, de préférence un appareil photo numérique. Il pèse de préférence moins de 3 kg, moins de 2 kg, moins de 1 kg, moins de 500 g, de préférence moins de 300 g.

L'appareil d'acquisition d'images 19 peut être intégré dans le support 12 ou, de préférence, être fixé provisoirement sur le support 12, grâce aux moyens de fixation 18 de l'appareil d'acquisition.

Dans un mode de réalisation préféré, l'appareil d'acquisition d'images comporte un module de traitement 59 configuré pour guider l'opérateur lors de la prise de vues, notamment pour qu'il ajuste de manière appropriée la longueur du support 12 et/ou qu'il positionne correctement sa bouche sur l'écarteur 14.

Dans un mode de réalisation préféré, le dispositif de prise de vues comporte un organe de détection 70 qu'un détecteur 72 de l'appareil d'acquisition d'images peut détecter (figure 5a).

De préférence, l'organe de détection 70 et le détecteur 72 sont configurés de manière que la détection ne soit possible que lorsque le dispositif de prise de vues est à proximité immédiate de l'appareil d'acquisition d'images. Avantageusement, l'appareil d'acquisition d'images ne réagit donc pas lorsque le dispositif de prise de vues en est éloigné. De préférence cependant, l'organe de détection 70 et le détecteur 72 sont configurés de manière que la détection soit possible à distance, de préférence à une distance supérieure à 5 cm, voire supérieure à 10 cm. Avantageusement, l'appareil d'acquisition d'images peut ainsi réagir très rapidement.

La position de l'organe de détection sur le support n'est pas limitative. De préférence cependant, il est disposé à moins de 5 cm, de préférence à moins de 3 cm, de préférence encore à moins de 2 cm du bord de l'ouverture d'acquisition. Avantageusement, la détection de l'appareil d'acquisition n'est ainsi réalisée que lorsque l'appareil d'acquisition d'images est rapproché de l'ouverture d'acquisition.

Dans un mode de réalisation préféré, l'organe de détection est un aimant et le détecteur 72 de l'appareil d'acquisition d'images comporte un magnétomètre. Un magnétomètre est capable de détecter une modification du champ magnétique qui l'entoure, et est donc capable de détecter l'approche d'un aimant.

Les téléphones mobiles étant généralement pourvus d'un magnétomètre, l'appareil d'acquisition d'images est de préférence un téléphone mobile.

Le détecteur 72 est configuré de manière à commander le module de traitement 59, en fonction de la détection de l'organe de détection 70.

Dans un mode de réalisation préféré, le détecteur 72 déclenche l'exécution du programme d'ordinateur chargé dans le module de traitement 59 en cas de détection de l'organe de détection 70. Il suffit donc à l'opérateur d'approcher du support l'appareil d'acquisition d'images, de préférence un téléphone, pour être guidé dans ses opérations d'acquisition d'images. Un tel mode de réalisation permet avantageusement une acquisition d'images par un opérateur qui n'a reçu aucune formation préalable.

De préférence, l'appareil d'acquisition d'images émet un message en réaction à la détection d'un organe de détection 70 par le détecteur 72. Le message est de préférence relatif à l'utilisation du dispositif de prise de vues. De préférence, l'appareil d'acquisition d'images est configuré pour émettre des messages relatifs à la fixation de l'appareil d'acquisition, par exemple au moyen d'un message vocal indiquant « positionnez votre appareil photos contre la face de couleur bleue du support », et/ou relatifs à la fixation de l'écarteur dentaire, par exemple en émettant un message vocal indiquant « pliez l'écarteur pour le fixer sur les pattes de couleur verte », et/ou relatifs à la chronologie des images actualisées à acquérir, par exemple en émettant un message vocal indiquant « prenez trois photos et envoyez-les à votre orthodontiste ».

Le message peut être purement informatif. Dans un mode de réalisation préféré, le message émis par l'appareil d'acquisition d'images dépend cependant des interactions entre l'opérateur et l'appareil d'acquisition d'images. Par exemple, l'appareil d'acquisition d'images peut émettre un message indiquant « l'appareil photos a été placé à l'envers » ou « l'appareil photos est mal orienté » ou « vos lèvres ne sont pas bien placées dans la goulotte de l'écarteur ».

L'organe de détection et le détecteur facilitent ainsi l'acquisition d'images actualisées.

De préférence, le détecteur 72 et/ou l'organe de détection 70 participent également à la fixation de l'appareil d'acquisition d'images sur le support. En particulier, l'organe de détection70 est de préférence un aimant 18. La mise en œuvre d'un aimant servant à la fois à la détection et à la fixation de l'appareil d'acquisition d'images est avantageusement simple, peu coûteuse et très pratique.

### Fonctionnement

Le fonctionnement du kit découle directement de la description qui précède.

Il est décrit pour le mode de réalisation représenté sur la figure 7.

Initialement, la plaque métallique 32 est de préférence fixée sur l'appareil d'acquisition d'images 19, en l'occurrence sous la coque de protection d'un téléphone mobile.

Dès que l'opérateur approche l'appareil d'acquisition d'images 19 de l'ouverture d'acquisition 26, la plaque métallique 32 est attirée par les aimants 18₁ et 18₂, et coopère avec ces derniers pour fixer l'appareil d'acquisition d'images dans une position prédéterminée, adaptée à l'acquisition d'images actualisées suivant un cadrage prédéterminé. En particulier, dans cette position, l'objectif de l'appareil d'acquisition d'images est correctement positionné par rapport à l'ouverture d'acquisition, de préférence sensiblement au centre de l'ouverture d'acquisition, et observe l'intérieur de la chambre 20. Dans cette position, l'appareil d'acquisition d'images 19 peut observer à la fois l'image directe et l'image réfléchie.

Par ailleurs, à l'approche du support, au moins un des aimants 18₁ et 18₂ du support est détecté par le magnétomètre du détecteur 72.

En conséquence, le magnétomètre lance automatiquement, c'est-à-dire sans intervention de l'opérateur, le programme d'ordinateur chargé dans le module de traitement 59 afin de guider l'opérateur dans ses opérations.

Si le boîtier est télescopique, l'opérateur règle alors la position, selon l'axe X, de la partie femelle par rapport à la partie mâle 12a, en fonction de l'appareil d'acquisition d'images 19 et de son réglage.

L'échelle disposée sur la partie mâle 12a du support et portant une marque pour chaque type d'appareil d'acquisition d'images facilite ce réglage.

De préférence, l'opérateur allume la source lumineuse 51 de manière à éclairer les dents et projeter sur les dents le repère, et en particulier une grille laser.

L'opérateur fixe également l'écarteur sur le support. Dans un mode de réalisation représenté, il insère une première des pattes droite et gauche derrière un premier des crochets droit et gauche, respectivement, puis plie légèrement l'écarteur pour autoriser l'insertion de la deuxième patte derrière le deuxième crochet. Lorsque l'opérateur relâche son action, l'écarteur tend à reprendre sa forme initiale, mais ce retour vers la forme initiale est entravé par les crochets. La pression de l'écarteur sur le support ainsi obtenu garantit le maintien en position de l'écarteur derrière les crochets.

De préférence, le module de traitement 59 est configuré pour commander l'acquisition automatique d'une ou plusieurs images actualisées et les analyser afin de détecter tout mauvais positionnement de l'appareil d'acquisition d'images et/ou de l'écarteur et/ou un éclairage défaillant et/ou une longueur du support inadaptée. De préférence encore, l'appareil d'acquisition d'images en informe instantanément l'opérateur afin qu'il corrige les défauts détectés.

Un dispositif selon l'invention permet ainsi de s'assurer d'un positionnement prédéterminé de l'appareil d'acquisition d'images par rapport à l'ouverture d'écarteur, et facilite donc l'acquisition d'images actualisées. Il permet d'obtenir des images actualisées de haute qualité, et en particulier de tailles sensiblement identiques, cadrées de la même façon et bien nettes. L'invention facilite donc l'analyse ultérieure des images actualisées.

Le patient, qui peut être également l'opérateur, dispose alors ses lèvres dans les goulottes définies par le rebord 34 de l'écarteur. Comme représenté sur la figure 3, les dents du patient sont alors bien dégagées. Le boîtier fermé masque avantageusement les dents, ce qui préserve l'intimité du patient.

Le dispositif est alors dans une position de service et l'appareil d'acquisition d'images 19 voit une image composée I_{c} du type de celle représentée sur la figure 3. Sur cette figure, l'image composée comporte une image directe I_{d} et 8 images réfléchies Iᵣ réfléchies par les différents miroirs 16 disposés dans la chambre 20.

En appuyant sur le déclencheur de l'appareil d'acquisition, l'opérateur acquiert l'image composée I_{c}. Avantageusement, il acquiert simultanément, comme dans le mode de réalisation de la figure 3, une vue de face (image directe I_{d}) et un ensemble de vues de biais (images réfléchies Iᵣ). Bien entendu, les images réfléchies sont inversées par rapport à la réalité. Par exemple, sur l'image réfléchie qui se trouve au-dessus de l'image directe I_{d}, l'arcade supérieure est représentée sous l'arcade inférieure.

Les images réfléchies peuvent avantageusement correspondre à des conditions d'acquisition dans lesquelles l'axe optique de l'appareil d'acquisition est fortement incliné par rapport au plan sagittal. Or l'utilisation de l'appareil d'acquisition dans ces conditions, en observation directe, c'est-à-dire sans miroir, étant souvent difficile pour le patient.

L'image composée est ensuite transmise au module de traitement 59, par des moyens filaires ou non filaires, par exemple par Wifi ou par Bluetooth ^{®}.

Le traitement d'images permet en particulier, suivant des méthodes de traitement conventionnelles, d'isoler l'image directe et la ou les images réfléchies. Dans un mode de réalisation, le traitement comporte également une opération d'inversion des images réfléchies et/ou une opération de correction des effets de perspective et/ou une opération de correction des couleurs au moyen de la mire colorimétrique 34.

L'analyse d'une des images directe et réfléchies, de préférence de l'image directe, permet de déterminer les conditions d'acquisition de ladite image, de préférence suivant l'enseignement de PCT/EP2015/074896, incorporé par référence. Les conditions d'acquisition des autres images peuvent avantageusement en être déduites, en tenant simplement compte de la géométrie du support 12. En particulier, la calibration de l'appareil d'acquisition est la même pour toutes les images directes et réfléchies. En outre, de simples considérations géométriques permettent de déterminer la position et l'orientation de l'appareil d'acquisition dans l'espace qui, en l'absence de miroir, auraient permis à l'appareil d'acquisition d'acquérir une image réfléchie.

Dans un mode de réalisation, le module de traitement 59, de préférence intégré dans l'appareil d'acquisition d'images 19, commande l'acquisition de plusieurs images composées dans des conditions d'acquisition différentes, et en particulier avec des distances focales différentes. Par exemple, des première, deuxième et troisième images composées peuvent être acquises en focalisant successivement l'appareil d'acquisition sur les incisives, sur les prémolaires et sur les molaires. Avantageusement, chaque dent est ainsi représentée de manière nette sur au moins une des images composées.

Dans un mode de réalisation, la prise de plusieurs images successives dans des conditions d'acquisition différentes résulte d'un unique actionnement du déclencheur de l'appareil d'acquisition d'images. En particulier, l'appareil d'acquisition d'images peut être configuré pour prendre successivement plusieurs photos, avec des distances focales différentes, en conséquence d'un unique déclenchement.

Par ailleurs, l'appareil d'acquisition acquiert de préférence une image des dents du patient représentant également les mires colorimétriques et de translucidité.

Le module de traitement 59, connaissant les propriétés de couleur et de translucidité réelles des mires, corrige alors l'image jusqu'à ce que les représentations desdites mires sur l'image présentent ces propriétés.

Pour que la correction soit précise, la source lumineuse 51 est de préférence réglée de manière qu'à l'instant de l'acquisition de l'image, les mires soient éclairées dans les mêmes conditions que lors de la mesure des propriétés réelles. L'utilisation d'un boîtier fermé facilite le contrôle de l'éclairage.

Toutes les images acquises par l'appareil d'acquisition sont ainsi avantageusement comparables, quel que soit l'environnement lumineux extérieur au dispositif au moment de leur acquisition.

Comme cela apparaît clairement à présent, l'invention facilite considérablement l'analyse des images actualisées.

Un dispositif selon le premier perfectionnement principal permet avantageusement une acquisition très rapide de plusieurs images, typiquement en moins d'une minute, sans avoir recours à une personne spécialisée, notamment un dentiste ou un orthodontiste. L'acquisition d'images peut être en particulier effectuée par le patient lui-même ou par un de ses proches, avec un simple téléphone portable, n'importe où, et en particulier en dehors d'un cabinet médical, dentaire ou d'orthodontie.

En outre, si la géométrie du support, et en particulier l'orientation et le positionnement d'un miroir, est connue, il suffit de déterminer les conditions d'acquisition de l'image directe pour pouvoir déterminer, par simple calcul, les conditions d'acquisition de l'image réfléchie par ce miroir. La mise en œuvre du procédé décrit dans PCT/EP2015/074896 en est considérablement accélérée.

Enfin, l'appareil d'acquisition 19 étant immobilisé par rapport aux dents, l'image composée est avantageusement nette et, si le dispositif comporte une source lumineuse contrôlée, bien contrastée.

Un dispositif selon le deuxième perfectionnement principal permet un suivi de l'évolution des propriétés d'aspect des dents. Il permet également une mesure précise de ces propriétés à tout instant, par le patient lui-même, et en particulier peu avant la réalisation d'une prothèse par exemple. La prothèse présente ainsi un aspect particulièrement proche de celui des dents du patient.

Un dispositif selon le troisième perfectionnement principal facilite le contrôle de l'éclairage lors de l'acquisition d'images actualisées et permet de préserver l'intimité du patient.

Un kit selon l'invention facilite considérablement l'utilisation d'un dispositif de prise de vues selon l'invention. En particulier, il permet, dans un mode de réalisation préféré, de guider automatiquement l'utilisateur dans ses opérations. La qualité des images actualisées en est avantageusement améliorée.

Bien entendu, l'invention n'est pas limitée aux modes de réalisation décrits et représentés, fournis à des fins illustratives seulement.

Notamment, la forme du boîtier n'est pas limitative.

En outre, bien que ce mode de réalisation ne soit pas préféré, la fixation de l'appareil d'acquisition d'images peut mettre en œuvre un ou plusieurs aimants fixés sur l'appareil d'acquisition d'images et une ou plusieurs pièces métalliques fixées sur le support.

Les pièces métalliques des moyens de fixation de l'appareil d'acquisition d'images peuvent être également remplacées, au moins partiellement, par des aimants agencés de manière à attirer les aimants fixés sur le support.

Le miroir n'est pas nécessairement plan. Il peut être en particulier configuré pour compenser des effets de perspective et/ou pour refléter des régions particulières de la bouche.

Le miroir peut être mobile par rapport au support, et en particulier peut être mobile en translation, notamment pour autoriser une modification de la distance entre le miroir d'une part et l'ouverture d'écarteur et/ou l'ouverture d'acquisition d'autre part.

Le miroir peut être également mobile en rotation, en particulier autour d'un axe de rotation perpendiculaire à l'axe X de l'écarteur, deux axes étant dits perpendiculaires lorsque deux plans orthogonaux à ces axes sont perpendiculaires l'un à l'autre.

De préférence, l'appareil d'acquisition d'images comporte un applicatif configuré pour guider l'opérateur afin qu'il positionne et oriente correctement le miroir. Dans un mode de réalisation, le positionnement et/ou l'orientation du miroir sont facilités par une échelle portant des indications relatives à différents appareils d'acquisition. L'opérateur peut ainsi facilement disposer le miroir en fonction de l'appareil d'acquisition utilisé.

Dans un mode de réalisation, le dispositif comporte un ou plusieurs actionneurs adaptés pour régler la longueur du support et/ou le positionnement et/ou l'orientation du miroir en fonction de consignes, de préférence en fonction de consignes reçues de l'appareil d'acquisition d'images 19.

Dans un mode de réalisation, le dispositif comporte également un ou plusieurs capteurs configurés pour mesurer la longueur du support 12 et/ou le positionnement et/ou l'orientation du miroir, et un émetteur capable de transmettre ladite mesure à l'appareil d'acquisition d'images. Avantageusement, une configuration optimale du dispositif peut être obtenue par une commande des actionneurs suivant une consigne, de préférence fournie par l'appareil d'acquisition d'images, ladite consigne étant de préférence fonction de l'image observée par l'appareil d'acquisition d'images et/ou par les mesures prises par les capteurs.

En outre, le nombre de miroirs n'est pas limité et plusieurs miroirs, de préférence tous les miroirs présentent de préférence une ou plusieurs des caractéristiques préférées du miroir 16 décrit précédemment. En particulier, un ou plusieurs des miroirs peuvent être pourvus d'un capteur, d'un actionneur et de moyens de communication avec l'appareil d'acquisition d'images, comme décrit précédemment.

## Revendications

1. Kit de prise de vues comportant
- un dispositif de prise de vues comportant :
- un support (12) ;
- un écarteur dentaire (14) fixé sur le support, et définissant une ouverture d'écarteur (24), l'écarteur comportant un rebord (34) s'étendant autour de l'ouverture d'écarteur et étant agencé de manière que les lèvres du patient puissent y reposer en laissant apparaître les dents du patient à travers ladite ouverture d'écarteur ; et
- des moyens de fixation (18) d'un appareil d'acquisition d'images (19) sur le support dans une position dans laquelle l'appareil d'acquisition est orienté pour recevoir une image de l'ouverture d'écarteur ;
le support définissant une chambre sensiblement fermée lorsque l'ouverture de l'écarteur et l'ouverture d'acquisition sont obturée ; et
- un dit appareil d'acquisition d'images (19) fixé sur le dispositif dans une position dans laquelle l'appareil d'acquisition est orienté pour recevoir une image de l'ouverture d'écarteur, l'appareil d'acquisition d'images étant un téléphone mobile.

2. Kit selon la revendication 1, comportant des moyens de fixation (15) de l'écarteur sur le support désactivables, qui maintiennent l'écarteur fléchi autour d'un axe Y sensiblement vertical dans la position de service.

3. Kit selon la revendication immédiatement précédente, dans lequel l'écarteur (14), d'axe X, comporte des oreilles (36) agencées de manière à écarter les joues des dents et/ou des pattes droite (38a) et gauche (38b) sensiblement perpendiculaires à l'axe X.

4. Kit selon l'une quelconque des deux revendications immédiatement précédentes, comportant des moyens de fixation (15) de l'écarteur sur le support choisis parmi des moyens de clipsage, des bandes auto-agrippantes de type Velcro^{®}, des mâchoires de serrage, des vis, et des aimants.

5. Kit selon l'une quelconque des revendications précédentes, dans lequel le support présente la forme d'un boîtier ne débouchant vers l'extérieur sensiblement que par l'ouverture d'écarteur et par une ouverture d'acquisition (26) à travers laquelle le téléphone mobile fixé sur le support reçoit ladite image de l'ouverture d'écarteur.

6. Kit selon l'une quelconque des revendications précédentes, dans lequel lesdits moyens de fixation (18) sont magnétiques et configurés de manière que le téléphone mobile ne puisse être fixé sur le support que dans une unique position prédéterminée.

7. Kit selon l'une quelconque des revendications précédentes, dans lequel le dispositif de prise de vues comporte un organe de détection (70) et le téléphone mobile comporte un détecteur (72) configuré de manière à détecter l'organe de détection lorsque l'organe de détection est à moins de 20 cm du détecteur.

8. Kit selon la revendication immédiatement précédente, dans lequel
le détecteur (72) est configuré de manière à ne détecter l'organe de détection que lorsque l'organe de détection est à moins de 20 cm du détecteur et/ou
l'organe de détection (70) et le détecteur (72) sont configurés de manière que la détection soit possible à une distance supérieure à 5 cm.

9. Kit selon l'une quelconque des deux revendications immédiatement précédentes, dans lequel l'organe de détection (70) est disposé à moins de 5 cm du bord de l'ouverture d'acquisition.

10. Kit selon l'une quelconque des trois revendications immédiatement précédentes, dans lequel le détecteur (72) comporte un magnétomètre.

11. Kit selon l'une quelconque des quatre revendications immédiatement précédentes, dans lequel le détecteur (72) est configuré pour déclencher une exécution d'un programme d'ordinateur chargé dans un module de traitement (59) du téléphone mobile en cas de détection de l'organe de détection (70).

12. Kit selon la revendication immédiatement précédente, dans lequel le module de traitement est configuré pour acquérir, en réaction à la détection de l'organe de détection (70) par le détecteur (72), une ou plusieurs images actualisées, puis les analyser afin de détecter un mauvais positionnement du téléphone mobile et/ou de l'écarteur et/ou un éclairage défaillant de l'ouverture d'écarteur et/ou une longueur du support inadaptée.

13. Kit selon l'une quelconque des six revendications immédiatement précédentes, dans lequel le téléphone mobile émet un message en réaction à la détection de l'organe de détection (70) par le détecteur (72), le message étant relatif à l'utilisation du dispositif de prise de vues et/ou relatif à la fixation du téléphone mobile et/ou relatif à la fixation de l'écarteur dentaire et/ou relatif à la chronologie des images actualisées à acquérir.

14. Kit selon l'une quelconque des sept revendications immédiatement précédentes, dans lequel lesdits moyens de fixation (18) comportent l'organe de détection (70).

15. Kit selon l'une quelconque des revendications précédentes, dans lequel le téléphone mobile est configuré pour prendre successivement plusieurs photos, avec des distances focales différentes, en conséquence d'un unique déclenchement.

## Patentansprüche

1. Aufnahmeset bestehend aus
- einer Bildaufnahmevorrichtung mit:
- einer Halterung (12);
- einem Mundspreizer (14), der an der Halterung befestigt ist und eine Spreizeröffnung (24) definiert, wobei der Spreizer einen Rand (34) aufweist, der sich um die Spreizeröffnung herum erstreckt und so angeordnet ist, dass die Lippen des Patienten darauf aufliegen können, wobei die Zähne des Patienten durch die Spreizeröffnung sichtbar sind; und
- Befestigungsmitteln (18) zum Befestigen eines Bilderfassungsgeräts (19) auf der Halterung in einer Position, in der das Erfassungsgerät so ausgerichtet ist, dass es ein Bild der Spreizeröffnung erhält;
wobei die Halterung eine im Wesentlichen geschlossene Kammer definiert, wenn die Spreizeröffnung und die Erfassungsöffnung verschlossen sind; und
- einem sogenannten Bilderfassungsgerät (19), das an der Vorrichtung in einer Position befestigt ist, in der das Bilderfassungsgerät so ausgerichtet ist, dass es ein Bild der Spreizeröffnung erhält, wobei das Bilderfassungsgerät ein Mobiltelefon ist.

2. Set nach Anspruch 1 mit deaktivierbaren Mitteln zur Befestigung (15) des Spreizers auf der Halterung, die den um eine im Wesentlichen vertikale Y-Achse gebogenen Spreizer in der Betriebsposition halten.

3. Set nach dem unmittelbar vorhergehenden Anspruch, wobei der Spreizer (14) der X-Achse Ohren (36) aufweist, die so angeordnet sind, dass sie die Wangen der Zähne und/oder die rechte (38a) und linke (38b) Lasche, die im Wesentlichen senkrecht zur X-Achse stehen, auseinander spreizen.

4. Set nach einem der beiden unmittelbar vorhergehenden Ansprüchen mit Mitteln zur Befestigung (15) des Spreizers auf der Halterung, ausgewählt aus Clip-Befestigungsmitteln, Klettverschlüssen vom Typ Velcro^{®}, Klemmbacken, Schrauben und Magneten.

5. Set nach einem der vorhergehenden Ansprüche, wobei die Halterung die Form eines Gehäuses aufweist, das im Wesentlichen nur durch die Spreizeröffnung und eine Erfassungsöffnung (26) nach außen offen ist, durch die das auf der Halterung befestigte Mobiltelefon das Bild der Spreizeröffnung empfängt.

6. Set nach einem der vorhergehenden Ansprüche, wobei die Befestigungsmittel (18) magnetisch sind und so konfiguriert sind, dass das Mobiltelefon nur in einer einzigen vorbestimmten Position an der Halterung befestigt werden kann.

7. Set nach einem der vorhergehenden Ansprüche, wobei die Bildaufnahmevorrichtung ein Detektionselement (70) aufweist und das Mobiltelefon einen Detektor (72) aufweist, der so konfiguriert ist, dass er das Detektionselement erkennt, wenn sich das Detektionselement weniger als 20 cm vom Detektor entfernt befindet.

8. Set nach dem unmittelbar vorhergehenden Anspruch, wobei
der Detektor (72) so konfiguriert ist, dass er das Detektionselement nur dann erkennt, wenn sich das Detektionselement weniger als 20 cm vom Detektor entfernt befindet, und/oder
das Detektionselement (70) und der Detektor (72) so konfiguriert sind, dass die Erfassung in einer Entfernung von mehr als 5 cm möglich ist.

9. Set nach einem der beiden unmittelbar vorhergehenden Ansprüche, wobei das Detektionselement (70) weniger als 5 cm vom Rand der Erfassungsöffnung entfernt angeordnet ist.

10. Set nach einem der drei unmittelbar vorhergehenden Ansprüche, wobei der Detektor (72) ein Magnetometer enthält.

11. Set nach einem der vier unmittelbar vorhergehenden Ansprüche, wobei der Detektor (72) so konfiguriert ist, dass er bei Erkennung des Detektionselements (70) die Ausführung eines in ein Verarbeitungsmodul (59) des Mobiltelefons geladenen Computerprogramms auslöst.

12. Set nach dem unmittelbar vorhergehenden Anspruch, wobei das Verarbeitungsmodul konfiguriert ist, als Reaktion auf die Erkennung des Detektionselements (70) durch den Detektor (72) ein oder mehrere aktualisierte Bilder zu erfassen und diese anschließend zu analysieren, um eine falsche Positionierung des Mobiltelefons und/oder des Spreizers und/oder eine fehlerhafte Beleuchtung der Spreizeröffnung und/oder eine ungeeignete Länge der Halterung zu erkennen.

13. Set nach einem der sechs unmittelbar vorhergehenden Ansprüche, wobei das Mobiltelefon als Reaktion auf die Erkennung des Detektionselements (70) durch den Detektor (72) eine Nachricht sendet, wobei sich die Nachricht auf die Verwendung der Bildaufnahmevorrichtung und/oder auf die Befestigung des Mobiltelefons und/oder auf die Befestigung des Mundspreizers und/oder auf die Chronologie der zu erfassenden aktualisierten Bilder bezieht.

14. Set nach einem der sieben unmittelbar vorhergehenden Ansprüche, wobei die Befestigungsmittel (18) das Detektionselement (70) enthalten.

15. Set nach einem der vorhergehenden Ansprüche, wobei das Mobiltelefon so konfiguriert ist, dass es nacheinander mehrere Fotos mit unterschiedlichen Brennweiten als Ergebnis eines einzigen Auslösevorgangs aufnimmt.

## Claims

1. Imaging kit including
- an imaging device including:
- a support (12);
- a dental spreader (14) fastened to the support, and defining a spreader opening (24), the spreader including a rim (34) extending around the spreader opening and being arranged in such a way that the patient's lips may rest on it, leaving the patient's teeth visible through said spreader opening; and
- means (18) for fastening an image acquisition apparatus (19) to the support in a position in which the acquisition apparatus is oriented so as to receive an image of the spreader opening;
the support defining a chamber substantially closed when the spreader opening and the acquisition opening are closed; and
- a said image acquisition apparatus (19) fastened to the device in a position in which the acquisition apparatus is oriented to receive an image of the spreader opening, the image acquisition apparatus being a mobile phone.

2. Kit according to Claim 1, including deactivatable means (15) for fastening the spreader to the support, which hold the spreader flexed about a substantially vertical Y axis in the service position.

3. Kit according to the immediately preceding claim, in which the spreader (14), of axis X, includes lobes (36) that are arranged so as to spread the cheeks away from the teeth and/or right (38a) and left (38b) tabs which are substantially perpendicular to the X axis.

4. Kit according to either one of the two immediately preceding claims, including means (15) for fastening the spreader to the support chosen from clip-fastening means, self-gripping strips of Velcro^{®} type, clamping jaws, screws, and magnets.

5. Kit according to any one of the preceding claims, in which the support takes the form of a box that is in communication with the outside substantially only via the spreader opening and via an acquisition opening (26) through which the mobile phone fastened to the support receives said image of the spreader opening.

6. Kit according to any one of the preceding claims, in which said fastening means (18) are magnetic and configured so that the mobile phone may be fastened to the support in only one predetermined position.

7. Kit according to any one of the preceding claims, in which the imaging device includes a detection member (70) and the mobile phone includes a detector (72) configured so as to detect the detection member when the detection member is less than 20 cm from the detector.

8. Kit according to the immediately preceding claim, in which
the detector (72) is configured so as to detect the detection member only when the detection member is less than 20 cm from the detector and/or
the detection member (70) and the detector (72) are configured in such a way that detection is possible at a distance greater than 5 cm.

9. Kit according to either one of the two immediately preceding claims, in which the detection member (70) is positioned less than 5 cm from the edge of the acquisition opening.

10. Kit according to any one of the three immediately preceding claims, in which the detector (72) includes a magnetometer.

11. Kit according to any one of the four immediately preceding claims, in which the detector (72) is configured to trigger an execution of a computer program loaded on a processing module (59) of the mobile phone in the event that the detection member (70) is detected.

12. Kit according to the immediately preceding claim, in which the processing module is configured to acquire, in response to the detection of the detection member (70) by the detector (72), one or more updated images, then analyse them to detect an incorrect positioning of the mobile phone and/or of the spreader and/or a poor illumination of the spreader opening and/or an unsuitable support length.

13. Kit according to any one of the six immediately preceding claims, in which the mobile phone transmits a message in response to the detection of the detection member (70) by the detector (72), the message relating to the use of the imaging device and/or relating to the fastening of the mobile phone and/or relating to the fastening of the dental spreader and/or relating to the timing of the updated images to be acquired.

14. Kit according to any one of the seven immediately preceding claims, in which said fastening means (18) include the detection member (70).

15. Kit according to any one of the preceding claims, in which the mobile phone is configured to take multiple photos in succession, with different focal lengths, as a result of a single trigger action.
